# EUROPÄISCHE PATENTANMELDUNG

(11) **EP 2 161 574 A2**
(43) Veröffentlichungstag der Anmeldung: **10.03.2010**
(21) Anmeldenummer: 09168742.6
(22) Anmeldetag: 26.08.2009
(51) Int. Cl.: G01N 33/28

(54) **Messanordnung zum Ermitteln des Ölzustands**

(30) Priorität: 05.09.2008 DE 102008045794
(71) Anmelder: Kleenoil Panolin AG, 79804 Dogern (DE)
(72) Erfinder: Krstic, Milorad, 79804, Dogern (DE)
(74) Vertreter: Kietzmann, Lutz

(57) **Zusammenfassung**

Die Erfindung betrifft eine Messanordnung zum Ermitteln des Zustands von Öl in einem Kreislauf, umfassend einen Sensor (1), welcher zumindest in einer Nebenleitung des Kreislaufs angeordnet ist und Parameter des Öls erfasst, und eine elektronische Auswerteinheit (3), die mit dem Sensor (1) verbunden ist und anhand von dessen erfassten Parametern Zustandsgrößen des Öls ermittelt, wobei die Auswerteinheit (3) im weiteren Verlauf mit einer Anzeigeeinheit (5) in Verbindung steht, über welche mittels drei optischer Anzeigemittel (6, 7, 8) der Zustand des Öls anzeigbar ist, von welchen ein erstes (6) grün, ein zweites (7) gelb und ein drittes (8) rot leuchtend ausgebildet ist.

## Beschreibung

### Gebiet der Erfindung

Die Erfindung betrifft eine Messanordnung zum Ermitteln des Zustands von Öl in einem Kreislauf, umfassend einen Sensor, welcher zumindest in einer Nebenleitung des Kreislaufs angeordnet ist und Parameter des Öls erfasst, und eine elektronische Auswerteinheit, die mit dem Sensor verbunden ist und anhand von dessen erfassten Parametern Zustandsgrößen des Öls ermittelt.

### Hintergrund der Erfindung

Im Bereich der Motoren, Getriebe und Maschinen sowie bei Hydraulikkreisläufen finden üblicherweise Öle als Schmier- oder Betriebsmittel Anwendung. Während des Einsatzes verändern sich jedoch aufgrund des Eintrags von Bestandteilen, wie beispielsweise Wasser, Russpartikel, sauerer Verbrennungsprodukte oder Partikel, die Eigenschaften des jeweils verwendeten Öls. Im Bereich der Verbrennungsmotoren enthalten die Öle zudem häufig Additive, um die Viskosität bei den vorherrschenden Temperaturen zu verbessern, welche sich allerdings mit der Zeit zersetzen. Aufgrund dieser Tatsachen ist es nötig, die verwendeten Öle ab einem bestimmten Zeitpunkt auszutauschen, um die geforderten Schmiereigenschaften oder Betriebseigenschaften zu garantieren. Üblicherweise erfolgt hierzu in bestimmten Zeitabständen ein Austausch des Öls. Da diese Wechselintervalle allerdings auf statistischen Werten beruhen, kann im Einzelfall der Austausch eines Öls erfolgen, welches noch zum Einsatz geeignet ist. Eine Möglichkeit, um dieser Tatsache und den steigenden Rohstoffkosten zu begegnen, ist, mit Sensoren den Zustand des Öls zu ermitteln und erst ab Unterschreiten bestimmter Grenzwerte einen Austausch des Öls vorzunehmen.

### Stand der Technik

Aus der DE 698 10 288 T2 ist eine derartige Messanordnung bekannt, bei welcher ein Sensor in einem Ölkreislauf angeordnet ist, um die Ölgüte zu bestimmen. Der Sensor ist hierbei nach Art eines Kapazitätssensors ausgeführt und bestimmt die Dielektrizitätskonstante des Öls. Im weiteren Verlauf ist der Sensor mit einer Auswerteinheit verbunden, welche die Signale des Sensors verarbeitet. Diese Auswerteinheit kann ferner eine Anzeigeeinheit aufweisen, welche den Ölzustand mittels einer Anzeige darstellt bzw. auf die Unterschreitung eines Grenzwertes mittels einer Warnlampe aufmerksam macht.

Nachteilhaft an einer derartigen Anordnung ist, dass die geschilderte Anzeigeeinheit kompliziert gehalten ist. Im Falle des Anzeigens der Ölparameter muss das Bedienpersonal Kenntnis davon haben, ab wann ein bestimmter Grenzbereich unterschritten wird. Bei Verwendung einer einfachen Warnlampe ist dagegen nicht der kontinuierliche Übergang vom zulässigen in den unzulässigen Bereich ersichtlich.

### Offenbarung der Erfindung

Es ist daher die Aufgabe der vorliegenden Erfindung, eine Messanordnung zum Ermitteln des Zustands von Öl in einem Kreislauf zu schaffen, welche dem Bediener auf einfache Art und Weise vermittelt, in welchem Zustand sich das Öl befindet.

Diese Aufgabe wird vorrichtungstechnisch ausgehend von Oberbegriff des Anspruchs 1 in Verbindung mit dessen kennzeichnenden Merkmalen gelöst. Aus verfahrenstechnischer Sicht findet eine Lösung dieser Aufgabe durch den Oberbegriff des nebengeordneten Anspruchs 8 in Verbindung mit dessen kennzeichnenden Merkmalen statt. Die jeweils darauf folgenden, abhängigen Ansprüche geben vorteilhafte Weiterbildungen der Erfindung wieder.

Die Erfindung umfasst die technische Lehre, dass die Auswerteinheit mit einer Anzeigeeinheit in Verbindung steht, über welche mittels drei optischer Anzeigemittel der Zustand des Öls anzeigbar ist. Von diesen drei Anzeigemitteln ist dabei ein erstes grün leuchtend, ein zweites gelb leuchtend und ein drittes rot leuchtend ausgebildet. Bei Betrieb der erfindungsgemäßen Messanordnung werden von einem Sensor erfasste Parameter mit Referenzwerten in der Auswerteinheit abgeglichen und eine Abweichung ermittelt. Diese ermittelte Abweichung wird in drei Abweichungskategorien eingeteilt, wobei anhand der Einteilung der jeweiligen Abweichung in eine dieser Kategorien die drei optischen Anzeigemittel nach Art einer Ampellogik betätigt werden. Eine derartige Messanordnung hat den Vorteil, dass es für den Bediener aufgrund der Ampellogik sehr leicht ersichtlich ist, wann das Öl zulässige Parameter aufweist bzw. ab wann eine Verschlechterung eingetreten ist oder es dringend ausgetauscht werden muss. Des Weiteren ist es aufgrund des Abgleichs in der Auswerteinheit mit einer hier hinterlegten Referenzkurve möglich, eine Änderung der Parameter des Öls zuverlässig zu erkennen.

Gemäß einer vorteilhaften Ausführungsform der Erfindung umfasst die Anzeigeeinheit einen Schalter. Durch Betätigen dieses Schalters werden die durch den Sensor erfassten Parameter des Öls und die in der Auswerteinheit hinterlegten Referenzwerte miteinander abgeglichen. Dies hat den Vorteil, dass somit eine Ermittlung des Zustands des Öls nur bei Betätigung des Schalters und folglich nur zu gewünschten Zeitpunkten erfolgt.

In Weiterbildung der Erfindung ist die Auswerteinheit mit der Anzeigeeinheit über ein Kabel verbunden. Dadurch kann eine sichere Datenübertragung zwischen der Auswerteinheit und der Anzeigeeinheit erfolgen.

Entsprechend einer weiteren, vorteilhaften Ausführungsform der Erfindung erfolgt eine Datenübertragung zwischen der Auswerteinheit und Anzeigeeinheit mittels GSM. Mittels des Mobilfunkstandards GSM können von der Auswerteinheit erfasste Parameter des Öls zu einer zentralen Stelle übermittelt werden, welche sich dementsprechend nicht unmittelbar im Bereich des Ölkreislaufs befinden muss. Folglich ist es auch möglich, die Sensoren mehrerer Ölkreisläufe von einer zentralen Überwachungsstelle zu kontrollieren.

In Weiterbildung der Erfindung ist der Sensor nach Art eines kapazitiven Sensors realisiert. Dies hat den Vorteil, dass der Zustand des Öls mit einer hohen Genauigkeit ermittelt werden kann.

Gemäß einer Ausgestaltung der Erfindung sind die Anzeigemittel der Anzeigeeinheit nach Art von Leuchtdioden ausgebildet. Dadurch wird eine energieeffiziente und gleichzeitig robuste Ausgestaltung der Anzeigeeinheit erreicht.

In Weiterbildung der Erfindung ist die Messanordnung in einem Ölkreislauf eines Kraftfahrzeuges vorgesehen, wobei die Auswerteinheit oder der Sensor mit einem Steuergerät einer Kraftfahrzeugssteuerungselektronik verbunden ist. Durch diese Maßnahme ist es möglich, dem Fahrzeugführer den Zustand des Öls im Bereich des Fahrzeugcockpits anzuzeigen und eventuell bei kritischen Ölparametern Einfluss auf die Motorsteuerung zu nehmen. Des Weiteren kann durch das Steuergerät somit eine Veränderung der Ölqualität über einen gewissen Zeitraum sowie im Falle eines Abgleichs mittels Schalterbetätigung die Häufigkeit einer derartigen Betätigung erfasst werden, wodurch Aussagen über die Güte der Wartungen des jeweiligen Kraftfahrzeuges getroffen werden können.

Gemäß einer vorteilhaften Ausführungsform der Erfindung wird im Falle der Betätigung das erste optische Anzeigemittel 3 Sekunden, das zweite 10 Sekunden und das dritte dauerhaft betätigt. Somit wird auf erste, größere Abweichungen zu den Referenzwerten durch ein längeres Leuchten des zweiten, gelben Anzeigemittels aufmerksam gemacht, während kritische Abweichungen sogar durch dauerhaftes Leuchten angezeigt werden. Folglich wird dadurch die Möglichkeit der Kenntnisnahme des Bedienpersonals über den Zustand des Öls weiter optimiert.

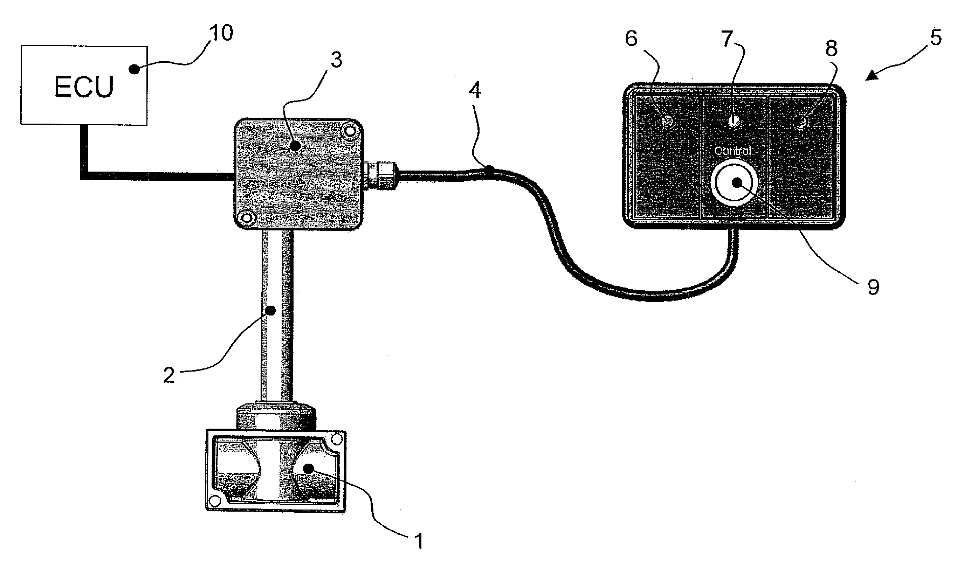

Weitere, die Erfindung verbessernde Maßnahmen werden nachstehend gemeinsam mit der Beschreibung eines bevorzugten Ausführungsbeispiels der Erfindung anhand der Figur näher dargestellt.

### Ausführliche Beschreibung der Ausführungsform

Die einzige Figur zeigt eine schematische Ansicht der erfindungsgemäßen Messanordnung, bei welcher ein Sensor 1 in einem hier nicht dargestellten Ölkreislauf angeordnet ist. In diesem Fall ist die Messanordnung derart gestaltet, dass der Sensor 1 in einem Vorratstank des Kreislaufs platziert werden kann. Dem Fachmann wird allerdings klar sein, dass daneben auch Platzierungen in einer Haupt- oder Nebenleitung des Kreislaufs denkbar wären. Zum Platzieren des Sensors 1 in dem - hier nicht dargestellten - Vorratstank steht dieser über ein Verbindungsrohr 2 mit einer Auswerteinheit 3 in Kontakt, welche anhand der vom Sensor 1 erfassten Parameter den Zustand des Öls ermittelt. Dazu ist in der Auswerteinheit 3 eine Referenzkurve des zu prüfenden Öls bei unterschiedlichen Temperaturen hinterlegt. Durch einen Vergleich der durch den Sensor 1 erfassten Parameter mit den in der Auswerteinheit 3 hinterlegten Referenzwerten wird daraufhin in der Auswerteinheit 3 eine Abweichung ermittelt. Im darauf folgenden Schritt wird diese ermittelte Abweichung einem von drei Abweichungsbereichen zugeordnet. Dabei fallen unter den ersten Abweichungsbereich Abweichungen, welche in einem zulässigen Rahmen liegen, d.h. dass Öl weist die noch nötigen Schmier- und Funktionseigenschaften auf und ist noch nicht in unzulässigem Maße mit Verunreinigungen kontaminiert. Im zweiten Abweichungsbereich weichen dagegen die durch den Sensor 1 ermittelten Werte stärker von den Referenzwerten ab, d.h. das Öl weist bereits eine verminderte Funktionsfähigkeit und/oder eine größere Verunreinigung auf und sollte in nächster Zeit getauscht werden. Der letzte Abweichungsbereich stellt dagegen extrem starke Abweichungen der durch den Sensor 1 erfassten Parameter von der Referenzkurve dar, was darauf hinweist, dass das Öl in seiner Funktionalität stark beeinträchtigt und mit Verunreinigungen kontaminiert ist. In diesem Fall sollte ein Ölwechsel umgehend erfolgen. Um den Zustand des Öls entsprechend der Einteilung der jeweiligen Abweichung in die Abweichungsbereiche darzustellen und dem Bedienpersonal anzuzeigen, ist die Auswerteinheit 3 über ein Kabel 4 mit einer Anzeigeeinheit 5 verbunden. Diese Anzeigeeinheit 5 verfügt über drei optische Anzeigemittel 6, 7 und 8 in Form von LED's, sowie einen Schalter 9. Dieser Schalter 9 ist dabei dafür vorgesehen, um bei Betätigung die Ermittlung der aktuellen Abweichung zwischen den Referenzwerten und den durch den Sensor 1 ermittelten Parametern des Öls in der Auswerteinheit 3 einzuleiten. Des Weiteren steht die Auswerteinheit 3 mit einem Steuergerät eines - hier ansonsten nicht dargestellten - Kraftfahrzeuges in Verbindung, um erfasste Parameter und Abweichungen auch einer Steuerelektronik des Kraftfahrzeuges zur Verfügung zu stellen.

Nach Betätigung des Schalters 9 wird entsprechend der Einteilung der Abweichung eines der optischen Anzeigemittel 6, 7 oder 8 gezielt betätigt. Liegt die Abweichung in dem ersten, noch zulässigen Abweichungsbereich, so wird dies durch eine Betätigung des ersten Anzeigemittels 6 durch grünes Leuchten angedeutet. Dabei leuchtet das Anzeigemittel 6 für 3 Sekunden auf. Bei sehr hohen ermittelten Abweichungen zwischen den erfassten Parametern und den Referenzwerten, welchen dem dritten Abweichungsbereich zuzuordnen und somit als kritisch einzustufen sind, wird das dritte optische Anzeigemittel 8 betätigt. Diese Abweichung wird dabei durch ein rotes Leuchten des dritten Anzeigemittels 8 angezeigt, wobei das Leuchten bestehen bleibt, bis es in der Auswerteinheit 3 zurückgesetzt wird, beispielsweise nach dem erfolgten Ölwechsel. Bei dazwischen liegenden Abweichungen, im zweiten Abweichungsbereich, wird das zweite Anzeigemittel 7 betätigt, welches daraufhin für 10 Sekunden gelb aufleuchtet. Hierdurch wird angedeutet, dass bereits größere Abweichungen zu den hinterlegten Referenzwerten bestehen und zeitnah ein Ölwechsel einzuplanen ist.

Folglich ist es durch die erfindungsgemäße Messanordnung möglich, durch Betätigung des Schalters 9 den Zustand des im Kreislauf vorhandenen Öls dem Bedienpersonal anzuzeigen. Aufgrund der Gestaltung der Anzeige nach Art einer Ampellogik können Parameterabweichungen des Öls direkt und einfach vom Bedienpersonal eingeschätzt werden. Zudem kann auf feste Ölwechselintervalle verzichtet werden, was den Rohstoff- und Betriebsaufwand einer Anlage entsprechend minimiert.

### Bezugszeichenliste

- 1: Sensor
- 2: Verbindungsrohr
- 3: Auswerteinheit
- 4: Kabel
- 5: Anzeigeeinheit
- 6: erstes optisches Anzeigemittel
- 7: zweites optisches Anzeigemittel
- 8: drittes optisches Anzeigemittel
- 9: Schalter
- 10: Steuergerät

## Patentansprüche

1. Messanordnung zum Ermitteln des Zustands von Öl in einem Kreislauf, umfassend einen Sensor (1), welcher zumindest in einer Nebenleitung des Kreislaufs angeordnet ist und Parameter des Öls erfasst, und eine elektronische Auswerteinheit (3), die mit dem Sensor (1) verbunden ist und anhand von dessen erfassten Parametern Zustandsgrößen des Öls ermittelt,
**dadurch gekennzeichnet, dass** die Auswerteinheit (3) im weiteren Verlauf mit einer Anzeigeeinheit (5) in Verbindung steht, über welche mittels drei optischer Anzeigemittel (6, 7, 8) der Zustand des Öls anzeigbar ist, von welchen ein erstes (6) grün, ein zweites (7) gelb und ein drittes (8) rot leuchtend ausgebildet ist.

2. Messanordnung nach Anspruch 1,
**dadurch gekennzeichnet, dass** die Anzeigeeinheit (5) einen Schalter (9) umfasst.

3. Messanordnung nach Anspruch 1 oder 2,
**dadurch gekennzeichnet, dass** die Auswerteinheit (3) mit der Anzeigeeinheit (5) über ein Kabel (4) verbunden ist.

4. Messanordnung nach Anspruch 1 oder 2,
**dadurch gekennzeichnet, dass** eine Datenübertragung zwischen der Auswerteinheit (3) und der Anzeigeeinheit (5) mittels GSM erfolgt.

5. Messanordnung nach einem der vorhergehenden Ansprüche,
**dadurch gekennzeichnet, dass** der Sensor (1) nach Art eines kapazitiven Sensors realisiert ist.

6. Messanordnung nach einem der vorhergehenden Ansprüche,
**dadurch gekennzeichnet, dass** die drei Anzeigemittel (6, 7, 8) nach Art von Leuchtdioden ausgebildet sind.

7. Kraftfahrzeug mit einem Ölkreislauf, umfassend eine Messanordnung nach einem der Ansprüche 1-6,
**dadurch gekennzeichnet, dass** die Auswerteinheit (3) oder der Sensor (1) mit einem Steuergerät (10) einer Kraftfahrzeugsteuerungselektronik verbunden ist.

8. Verfahren zum Ermitteln des Zustands von Öl in einem Kreislauf mit einer Messanordnung nach einem der Ansprüche 1-6, umfassend folgende Schritte:
- Abgleich der durch den Sensor (1) erfassten Parameter mit Referenzwerten in der Auswerteinheit (3) und Ermittlung einer Abweichung,
- Zuordnen der Abweichung einem von drei Abweichungsbereichen, und
- Betätigen eines der drei optischen Anzeigemittel (6, 7, 8) in Abhängigkeit des ermittelten Abweichungsbereich nach Art einer Ampellogik.

9. Verfahren nach Anspruch 8,
**dadurch gekennzeichnet, dass** im Falle der Betätigung das erste optische Anzeigemittel (6) 3 Sekunden, das zweite (7) 10 Sekunden und das dritte (8) dauerhaft betätigt wird.

10. Verfahren nach einem der Ansprüche 9-11,
**dadurch gekennzeichnet, dass** die erfassten Parametern und die Referenzwerten nur bei Betätigung des Schalters (9) miteinander abgeglichen werden.
